# EUROPEAN PATENT APPLICATION

(11) **EP 1 258 494 A1**
(43) Date of publication of application: **20.11.2002**
(21) Application number: 01130253.6
(22) Date of filing: 20.12.2001
(51) Int. Cl.: C07K 14/39, C07K 14/47, C12N 15/11, C12N 15/62

(54) **Multiprotein complexes from eukaryotes**

(30) Priority: 15.05.2001 EP 01111774
(71) Applicant: CELLZOME AG, 69117 Heidelberg (DE)
(72) Inventor: Bauer, Andreas Dr., 69123 Heidelberg (DE); Gavin, Anne-Claude Dr., 69181 Leimen (DE); Grandi, Paola Dr., 69117 Heidelberg (DE); Krause, Roland, 69115 Heidelberg (DE); Kruse, Ulrich Dr, 69221 Heidelberg (DE); Küster, Bernhard Dr, 69254 Malsch (DE); Marzioch, Martina, 69126 Heidelberg (DE); Schultz, Jörg Dr, 69126 Heidelberg (DE); Superti-Furga Giulio Dr, 69118 Heidelberg (DE)
(74) Representative: Grünecker, Kinkeldey, Stockmair & Schwanhäusser Anwaltssozietät

(57) **Abstract**

The present invention relates to multiprotein complexes from eukaryotes. The complexes are obtainable by using a protein as a bait and isolating the set of proteins which is attached thereto from cells. Such protein complexes may comprise up to 30 distinct proteins.

## Description

With reference to the decision of the President of the European Patent Office dated 9 June 2000, the full version of this document will be published in electronic form only.

Conformément à la décision du Président de l'Office européen des brevets, en date du 9 juin 2000, le document complet ne sera publié que sous forme électronique.

Mit Bezug auf den Beschluß des Präsidenten des Europäischen Patentamts vom 9. Juni 2000, wird die vollständige Fassung dieses Dokuments nur elektronisch veröffentlicht.

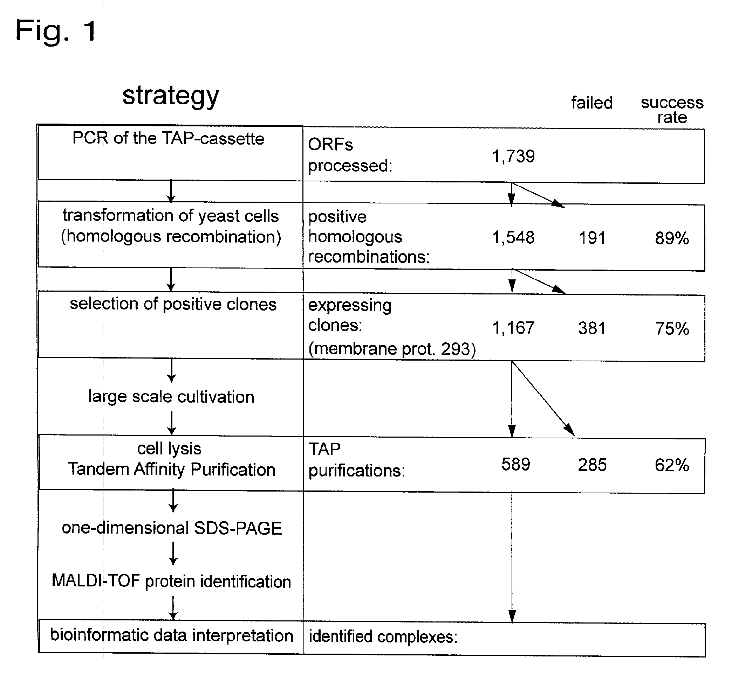

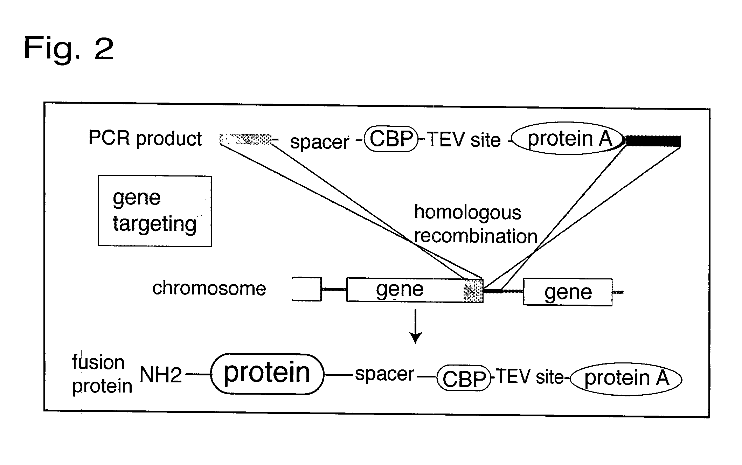

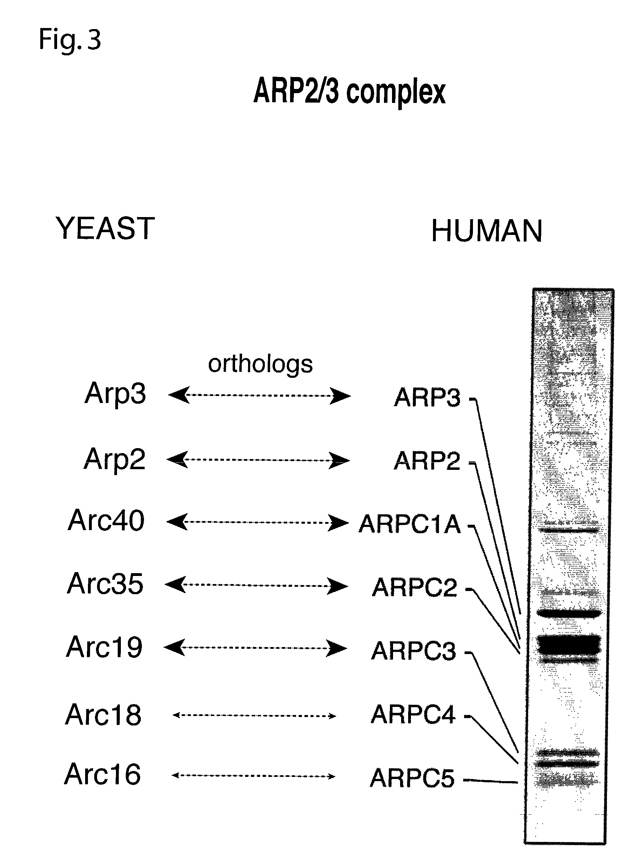

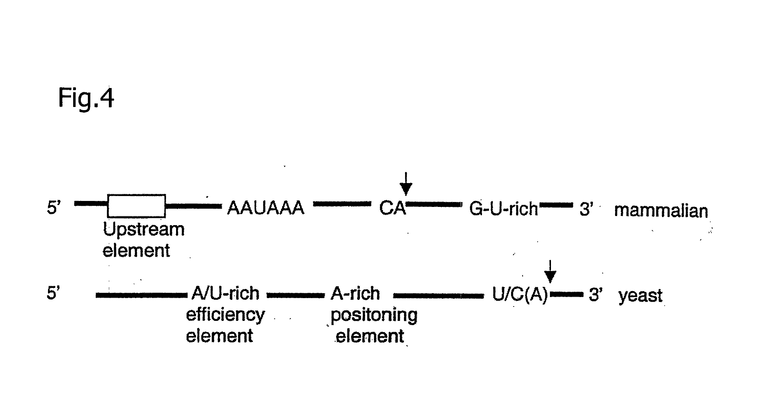

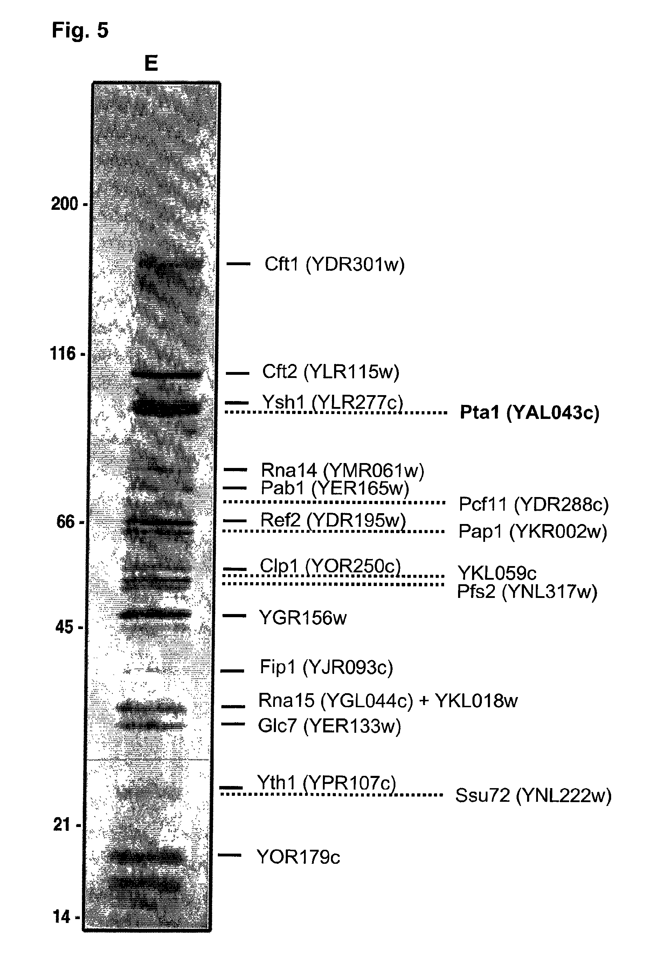

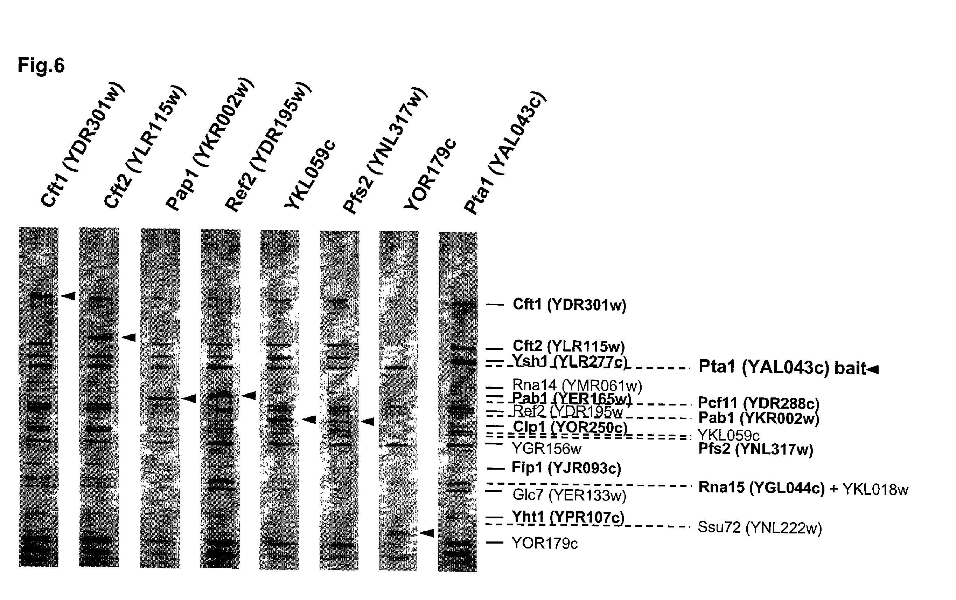

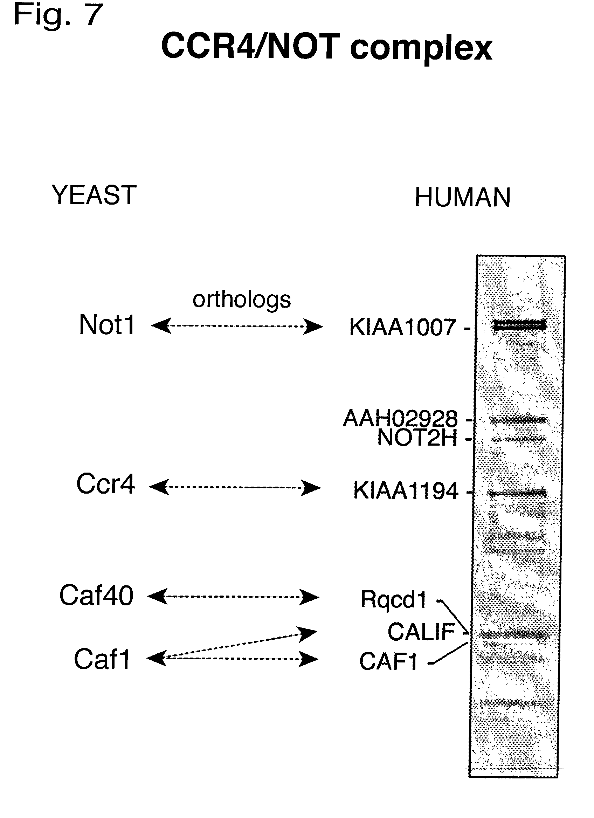

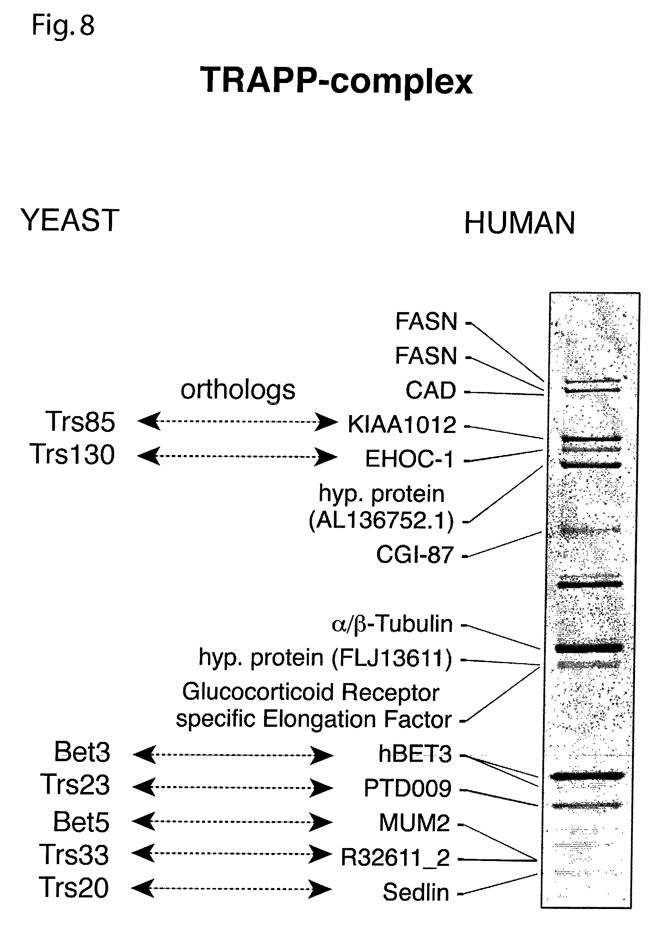

## Claims

1. An isolated complex selected from complex (I) and comprising
(a) a first protein, or a functionally active fragment or functionally active derivative thereof, which first protein is selected from the group of proteins in table 2, column A of a given complex, or a mammalian homolog thereof, or a variant of said protein encoded by a nucleic acid that hybridises to the nucleic acid of said protein or its complement under low stringency conditions; and
(b) a second protein, or a functionally active fragment or functionally active derivative thereof, which second protein is selected from the group of proteins in table 2, column B of a said complex, or a mammalian homolog thereof, or a variant of said second protein encoded by a nucleic acid that hybridises to the nucleic acid of said protein or its complement under low stringency conditions, wherein said first and said second protein are members of a native cellular complex, and wherein said low stringency conditions comprise hybridization in a buffer comprising 35% formamide, 5X SSC, 50 mM Tris-HCI (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1 % SDS for 1.5 hours at 55°C, and washing in a buffer consisting of 2X SSC, 25 mM Tris-HCI (pH 7.4), 5 mM EDTA, and 0.1% SDS for 1.5 hours at 60°C, and a complex (II) comprising at least two second proteins.

2. An isolated complex comprising all proteins in columns A and B of table 2 of a given complex, or the mammalian homologs thereof, or variants of said proteins encoded by nucleic acid that hybridises to the nucleic acid of any of said proteins or its complements under low stringency conditions, wherein proteins are members of a native cellular complex, and wherein said low stringency conditions comprise hybridization in a buffer comprising 35% formamide, 5X SSC, 50 mM Tris-HCI (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1 % SDS for 1.5 hours at 55°C, and washing in a buffer consisting of 2X SSC, 25 mM Tris-HCI (pH 7.4), 5 mM EDTA, and 0.1% SDS for 1.5 hours at 60°C.

3. An isolated complex that comprises all but 2 up to (n minus 1) proteins of all proteins in columns A and B of table 2 of a given complex , or the mammalian homologs thereof, or variants of said proteins or encoded by nucleic acid that hybridises to the nucleic acid of any of said proteins or its complements under low stringency conditions, wherein proteins are members of a native cellular complex, and wherein said low stringency conditions comprise hybridization in a buffer comprising 35% formamide, 5X SSC, 50 mM Tris-HCI (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1 % SDS for 1.5 hours at 55°C, and washing in a buffer consisting of 2X SSC, 25 mM Tris-HCI (pH 7.4), 5 mM EDTA, and 0.1% SDS for 1.5 hours at 60°C.

4. The complex according to claim 3, which comprises all but 1 of the all proteins in column A of table 2 of a given complex and column B of table 2 of said complex.

5. The complex of No. 1,2,3,4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.

6. The complex of claim 1, 2, 3, 4 comprising a fragment of said first protein and/or a fragment of said second protein, which fragment binds to another protein component of said complex.

7. The complex of claim 1, 2, 3, 4, 5, 6 that is involved in the biochemical activity as stated in column C of table 2.

8. The complex of claim 5, wherein the functionally active derivative is a fusion protein comprising said first protein or said second protein fused to an affinity tag or label.

9. An antibody or a fragment of said antibody containing the binding domain thereof, which binds the complex of claim 1, 2, 3, 4, 5, 6, 7 and which does not bind the first protein when uncomplexed or the second protein when uncomplexed.

10. A pharmaceutical composition comprising the protein complex of claim 1, 2, 3, 4, 5, 6, 7 or 8; and a pharmaceutically acceptable carrier.

11. A method for screening for a molecule that modulates directly or indirectly the function, activity or formation of the complex of any one of claim 1 - 8 comprising the steps of:
(a) exposing said complex, or a cell or organism containing said complex to one or more candidate molecules; and
(b) determining the amount of, the biochemical activity as stated in column C of table 2 of, or protein components of, said complex, wherein a change in said amount, activity, or protein components relative to said amount, activity or protein components in the absence of said candidate molecules indicates that the molecules modulate function, activity or composition of said complex.

12. The method of claim 11, wherein the amount of said complex is determined.

13. The method of claim 11, wherein the activity of said complex is determined.

14. The method of claim 13, wherein said determining step comprises isolating from the cell or organism said complex to produce said isolated complex and contacting said isolating complex with the substrate under conditions conducive to binding to the complex.

15. The method of claim 11, wherein the protein components of said complex are determined.

16. The method of claim 15, wherein said determining step comprises determining whether any of the proteins listed in column B of table 2 of said complex or the mammalian homologs thereof, or variant of said proteins encoded by a nucleic acid that hybridises to the nucleic acids of any of said proteins or its complements under low stringency conditions, is present in the complex, wherein said low stringency conditions comprise hybridization in a buffer comprising 35% formamide, 5X SSC, 50 mM Tris-HCI (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1 % SDS for 1.5 hours at 55°C, and washing in a buffer consisting of 2X SSC, 25 mM Tris-HCI (pH 7.4), 5 mM EDTA, and 0.1% SDS for 1.5 hours at 60°C.

17. The method of claim 11, wherein said method is a method of screening for a drug for treatment or prevention of diseases and disorders, preferably diseases or disorders selected from the diseases or disorders as listed in table 4 for said complex.

18. A method for screening for a molecule that binds the complex of anyone of claim 1 - 8 comprising the following steps:
(a) exposing said complex, or a cell or organism containing said complex, to one or more candidate molecules; and
(b) determining whether said complex is bound by any of said candidate molecules.

19. A method for diagnosing or screening for the presence of a disease or disorder or a predisposition for developing a disease or disorder in a subject, which disease or disorder is **characterized by** an aberrant amount of, biochemical activity as stated in column C of table 2 of, or component composition of, the complex of any one of the claim 1-8, comprising determining the amount of, biochemical activity as stated in column C of table 2 of, or protein components of, said complex in a sample derived from a subject, wherein a difference in said amount, activity, or protein components of, said complex in an analogous sample from a subject not having the disease or disorder or predisposition indicates the presence in the subject of the disease or disorder or predisposition.

20. The method of claim 19, wherein the amount of said complex is determined.

21. The method of claim 19, wherein the activity of said complex is determined.

22. The method of claim 21, wherein said determining step comprises isolating from the cell or organism said complex to produce said isolated complex and contacting said isolating complex with the substrate under conditions conducive to binding to the complex.

23. The method of claim 19, wherein the protein components of said complex are determined.

24. The method of claim 23, wherein said determining step comprises determining whether any of the proteins listed in column B of table 2 of said complex or the mammalian homologs thereof, or variant of said proteins encoded by a nucleic acid that hybridises to the nucleic acids of any of said proteins or its complements under low stringency conditions, is present in the complex, wherein said low stringency conditions comprise hybridization in a buffer comprising 35% formamide, 5X SSC, 50 mM Tris-HCI (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1 % SDS for 1.5 hours at 55°C, and washing in a buffer consisting of 2X SSC, 25 mM Tris-HCI (pH 7.4), 5 mM EDTA, and 0.1% SDS for 1.5 hours at 60°C.

25. A method for treating or preventing a disease or disorder **characterized by** an aberrant amount of, biochemical activity as stated in column C of table 2 of, or component composition of, the complex of anyone of claim 1- 8, comprising administering to a subject in need of such treatment or prevention a therapeutically effective amount of one or more molecules that modulate the amount of, biochemical activity as stated in column C of table of, or protein components of, said complex.

26. The method according to claim 25, wherein said disease or disorder involves decreased levels of the amount or activity of said complex.

27. The method according to claim 25, wherein said disease or disorder involves increased levels of the amount or activity of said complex.

28. Use of a molecule that modulates the amount of, biochemical activity as stated in column C of table of, or protein components of the complex of any one of claims 1-8 for the manufacture of a medicament for the treatment or prevention of a disease or disorder, preferably diseases or disorders selected from the diseases or disorders as listed in table 4 for said complex.

29. A kit comprising in one or more containers
(a) an isolated first protein, or a functionally active fragment or functionally active derivative thereof selected from the proteins in column A of table 2 of a given complex or a mammalian homolog thereof, or a variant of said protein encoded by a nucleic acid that hybridises to the nucleic acid of said protein or its complement under low stringency conditions; and
(b) an isolated second protein, or a functionally active fragment or functionally active derivative thereof selected from the proteins in column B of table 2 of a given complex or a mammalian homolog thereof, or a variant of said protein encoded by a nucleic acid that hybridises to the nucleic acid of said protein or its complement under low stringency conditions, wherein said first and said second protein are members of a native cellular complex, and wherein said low stringency conditions comprise hybridization in a buffer comprising 35% formamide, 5X SSC, 50 mM Tris-HCI (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1 % SDS for 1.5 hours at 55°C, and washing in a buffer consisting of 2X SSC, 25 mM Tris-HCI (pH 7.4), 5 mM EDTA, and 0.1% SDS for 1.5 hours at 60°C.

30. A kit comprising in a container the isolated complex of any one of claim 1-8 or the antibody of claim 9.

31. The complex of any one of claim 1-8, or the antibody or fragment of claim 9, for use in a method of diagnosing a disease or disorder, preferably diseases or disorders selected from the diseases or disorders as listed in table 4 for said complex.

32. A method for the production of a pharmaceutical composition comprising carrying-out the method of claim 11 or 18 to identify a molecule that modulates the function, activity or formation of said complex, and further comprising mixing the identified molecule with a pharmaceutically acceptable carrier.

33. A process for preparing complex of claim 1-8 and optionally the components thereof comprising the following steps:
expressing such a protein in a target cell,
isolating the protein complex which is attached to the tagged protein, and
optionally disassociating the protein complex and isolating the individual complex members.

34. The process according to claim 33 **characterized in that** the tagged protein comprises two different tags which allow two separate affinity purification steps.

35. The process according to any of claim 33-34, **characterized in that** two tags are separated by a cleavage site for a protease.

36. Component of the said complex obtainable by a process according to any of claims 33-35.

37. Complex of claim 1-8 and/or protein thereof as a target for an active agent of a pharmaceutical, preferably a drug target in the treatment or prevention of disease or disorder, preferably diseases or disorders selected from the diseases or disorders as listed in table 4 for said complex.

38. Protein of the complexes as listed in table 2 selected from
a) yeast proteins as listed in column D of table 2 for said complex;
b) the mammalian homologs/orthologs of the proteins of a);
c) derivatives of the above proteins according to a) and b) carrying one or more amino acid substitutions, deletions and/or additions; and
d) proteins showing a percent identity of at least 40% to any of the proteins of a) or b).

39. Components as described in claim 38, **characterized in that** it shows a percent identity of at least 70%, more preferably at least 90% to any of the above proteins of a) or b).

40. Components as described in claims 38, 39, **characterized in that** it is the human ortholog of any of the yeast proteins of the complex as listed in column D of table 2 for said complex.

41. Component as described above, **characterized in that** it is encoded by a nucleic acid sequence which hybridises to an amino acid encoding any of the yeast proteins of the complex as listed in column D of table 2 for said complex.
